# EUROPEAN PATENT SPECIFICATION

(11) **EP 4 369 909 B1**
(45) Date of publication and mention of the grant of the patent: **26.08.2026**
(21) Application number: 22751059.1
(22) Date of filing: 14.07.2022
(51) Int. Cl.: A01H 1/00, A01N 33/00, A61K 8/92, A61K 38/16

(54) **SYNERGISTIC ANTIMICROBIAL COMPOSITIONS CONTAINING SELECTED PEPTIDES AND FATTY ACIDS**
SYNERGISTISCHE ANTIMIKROBIELLE ZUSAMMENSETZUNGEN, WELCHE AUSGEWÄHLTE PEPTIDE UND FETTSÄUREN ENTHALTEN
COMPOSITIONS ANTIMICROBIENS SYNERGIQUES COMPRENANT DES PEPTIDES ET DES ACIDES GRAS SELECTIONNÉS

(30) Priority: 14.07.2021 IT 202100018530
(43) Date of publication of application: 22.05.2024
(73) Proprietor: CLEVER BIOSCIENCE S.R.L., 27062 Campospinoso Albaredo (PV) (IT)
(72) Inventor: MAZZEI, Emma, 20141 Milano (IT); BREVIARIO, Elisa, 24040 Bonate Sopra BG (IT); ZUCCHINALI, Stefano, 24040 Pontirolo Nuovo BG (IT); FRESCHI, Giorgio, 29121 Piacenza (IT)
(74) Representative: M. Zardi & Co S.A.
(86) International application number: PCT/EP2022/069722
(87) International publication number: WO 2023/285590

(56) References cited:
- WO-A1-2009/094719
- WO-A1-2010/015024
- WO-A1-2013/192190
- WO-A1-2015/195677
- WO-A1-2020/017980
- WO-A1-2020/035483
- CN-A- 108 992 368
- JP-A- H09 124 504
- KARNY AVISHAI ET AL: "Therapeutic nanoparticles penetrate leaves and deliver nutrients to agricultural crops", SCIENTIFIC REPORTS, vol. 8, no. 1, 1 December 2018 (2018-12-01), pages 7589, XP055830608, Retrieved from the Internet <URL:https://www.nature.com/articles/s41598-018-25197-y.pdf> DOI: 10.1038/s41598-018-25197-y
- HUANG C M ET AL: "Eradication of drug resistant Staphylococcus aureus by liposomal oleic acids", BIOMATERIALS, ELSEVIER, AMSTERDAM, NL, vol. 32, no. 1, 1 January 2011 (2011-01-01), pages 214 - 221, XP027493706, ISSN: 0142-9612, [retrieved on 20100928], DOI: 10.1016/J.BIOMATERIALS.2010.08.076
- HOBBY CHELSEA R. ET AL: "Exogenous fatty acids alter phospholipid composition, membrane permeability, capacity for biofilm formation, and antimicrobial peptide susceptibility in Klebsiella pneumoniae", vol. 8, no. 2, 1 February 2019 (2019-02-01), pages e00635, XP055896836, ISSN: 2045-8827, Retrieved from the Internet <URL:https://www.ncbi.nlm.nih.gov/pmc/articles/PMC6391273/pdf/MBO3-8-e00635.pdf> DOI: 10.1002/mbo3.635
- BROGDEN N. K. ET AL: "The Emerging Role of Peptides and Lipids as Antimicrobial Epidermal Barriers and Modulators of Local Inflammation", SKIN PHARMACOLOGY AND PHYSIOLOGY, vol. 25, no. 4, 1 January 2012 (2012-01-01), CH, pages 167 - 181, XP055902960, ISSN: 1660-5527, DOI: 10.1159/000337927
- YAN JUAN ET AL: "Plant antifungal proteins and their applications in agriculture", vol. 99, no. 12, 15 May 2015 (2015-05-15), Berlin/Heidelberg, pages 4961 - 4981, XP055902964, ISSN: 0175-7598, Retrieved from the Internet <URL:http://link.springer.com/article/10.1007/s00253-015-6654-6/fulltext.html> DOI: 10.1007/s00253-015-6654-6
- JOSHUA JACKMAN ET AL: "Nanotechnology Formulations for Antibacterial Free Fatty Acids and Monoglycerides", MOLECULES, vol. 21, no. 3, 3 March 2016 (2016-03-03), pages 305, XP055474421, DOI: 10.3390/molecules21030305

## Description

### FIELD OF THE INVENTION

The present invention pertains the field of antimicrobial products and compositions containing the same, for the use in the agronomic field.

### PRIOR ART

Many plants have an advantageous, often essential, relationship with the microorganisms of the environment (water, soil, air). However, this relationship may become unbalanced: in this case, the microorganisms, in particular fungi and bacteria, transform into parasites and kill the plants by depriving them of nutritious substances. For example, fungal infections destroy every year more than 125 million tons of crops worldwide.

These contaminations are mainly dealt with antimicrobial synthetic products; however, the latter entail many problems: they kill also microorganisms that are beneficial for the soil, thereby causing serious consequences for plants. Moreover, the synthetic antimicrobial agents cause several environmental effects, in addition to toxicological problems for humans who come in contact with them. These problems are particularly relevant and urgent in the agronomic field, considering the wide surfaces to be treated and the corresponding massive use of antimicrobial agents, which is required to ensure sufficient product concentrations in the vicinity of all the plants to be treated. Further problems are those related to the appearance of resistant strains, with the consequent growing need of newer antimicrobial products with high efficacy.

In recent years, the regulations for environment protection have imposed limits to the use of synthetic antimicrobial agents, supporting at the same time the research of new products with natural characteristics, which should be less toxic for humans and easier to dispose of. The use of antimicrobial peptides, i.e. small protein molecules consisting of 10-100 amino acids, broadly occurring in nature (in bacteria, plants, insects, etc.) is very interesting. Currently, about 800 substances classified as antimicrobial peptides are known. The first to be studied were cecropins, isolated from silkworm (Hyalophora cecropia) in the early 1980s, and melittin, isolated from the venom of honeybee (Apis mellifera). The latter is one of the peptides studied most thoroughly and is therefore often used as a reference for studying new molecules. The skin of several amphibian species is a rich source of peptides (bombesins, magainins, temporins, etc.), produced and secreted by granular glands in response to a variety of stimuli. In humans and in other mammals (mouse, rat, rabbit), antimicrobial peptides belonging to the defensin family are stored in the form of granules in neutrophils (blood cells specialized in phagocytosis), whereas polymorphonuclear leukocytes of bovines are rich in peptides belonging to the cathelicidin family that showed in vitro and in vivo a significant antimicrobial activity.

Antimicrobial peptides have an action spectrum that is quite aspecific and thus generally broad against viruses, bacteria, fungi and protozoans; the activity arises rapidly and extends to microorganisms that have developed resistance. The action mechanism is attributable to the alteration of cellular membranes, with effects such as disorganization of membrane structure, alteration of permeability, outflow of cytoplasm components and cell lysis (destruction). Some peptides, such as buforin, directly interact with intracellular targets (DNA and/or RNA) inhibiting functions that are vital for the cell. Other peptides (for example those derived from cathelicidins and defensins) inhibit pro-inflammatory and immune-defense response of the host organism.
The patent application WO 2009/094719A1 describes an association of a defensin and a fungicide in a compostion for plant protection. WO 2010/015024 describes an association of a defensin and a proteinase inhibitor in a compostion for plant protection. WO 2020/035483A1 describes an antimicrobial liquid crystal composition for coating surfaces comprising amphiphilic lipids and antimicrobial agents. WO 2020/017980A1 describes an ingestible formulation comprising a bioactive peptide in the form of thickened gel or edible oil. JPH09124504A describes a pharmaceutical antimicrobial peptide composition in emulsion form. The publication HOBBY CHELSEA R. ET AL: MICROBIOLOGYOPEN, vol. 8, no. 2, 1 February 2019, page e00635 describes the effect of exogenous fatty acids in altering the phospholipid composition of the microbial membrane and its susceptibility to antimicrobial peptides. The publication BROGDEN N. K. ET AL: SKIN PHARMACOLOGY AND PHYSIOLOGY, vol. 25, no. 4, 1 January 2012, pages 167-181 describes the role of peptides and lipids as antimicrobial epidermal barriers and modulators of local Inflammation. The publication YAN JUAN ET AL: APPLIED MICROBIOLOGY AND BIOTECHNOLOGY, vol. 99, no. 12, 15 May 2015, pages 4961-4981 describes proteins with plant antifungal activity and their applications in agriculture. The publication JOSHUA JACKMAN ET AL: MOLECULES, vol. 21, no. 3, 3 March 2016, page 305 describes nanotechnology formulations for antibacterial free fatty acids and monoglycerides.

The low selectivity of these compounds on one hand widens the action spectrum; on the other hand, it involves a non-tailored interaction with the concerned microorganism, resulting in significant variations of potency across different microorganisms, making it difficult to maintain an average high level of activity against a large group of target microorganisms. A particularly resistant sub-group of microorganisms is that of phytoplasma, i.e. special bacteria without cell wall: they access the inner parts of the plant (phloema) via vector insects and cause serious diseases, even lethal for the plant; to date there are no curative strategies to contrast phytoplasma: in fact, the traditional antibacterial strategies, aimed to hit the bacterial cell wall, are ineffective on these microorganisms and the available treatments are limited to preventive ones.
The low specificity of antimicrobial peptides could also increase the risk of undesired effects on the plant and/or on humans. The possibility of reducing the amount/concentration of these products is not effectively viable since this is associated to an undesired reduction of treatment efficacy. Therefore, there is still the need for new antimicrobial products and compositions that associate the advantage of a broad action spectrum to the advantage of a stronger activity, such as to allow the use in amounts lower than the standard ones without compromising the extent of the effect. In particular, there is still the need for synergistic compositions that allow to obtain a higher antimicrobial effect than the sum of those obtainable by the components constituting the composition, taken separately. These problems have been addressed by the present inventors and unexpectedly solved in accordance with the following disclosures.

### SUMMARY

It has been now discovered that it is possible to obtain an unexpectedly high antimicrobial activity by combining antimicrobial peptides and fatty acids. In particular, the antimicrobial peptides are selected in the classes of defensins. The fatty acids are selected from pelargonic acid, crotonic acid, caproleic acid, or mixtures thereof. The present peptides and fatty acids synergize, thereby providing a strong antimicrobial, in particular antifungal and antibacterial, activity, with important applications, especially in the agronomic field. Selected combinations of fatty acids with said antimicrobial peptides are also disclosed herein with additional advantages.

### DETAILED DESCRIPTION OF THE INVENTION

In the compositions of the invention, the above-mentioned ingredients are suitably formulated with excipients and a suitable carrier, in particular for the use in agriculture.

Defensins are a phylogenetically very old peptide family, with a highly conserved structure, that are present in mammals, insects and plants: they are amphipathic peptides capable of inserting in membranes and of inducing the pore formation resulting in death due to cells lysis. There are two main defensin categories: α and β and they differ in the type of producing cell and thus in localization. α defensins are mainly produced by neutrophils (contained in primary granules) and by Paneth cells; they are produced and secreted as an inactive form of pro-peptide and are activated by proteolytic cleavage by trypsin. β defensins are produced by epithelial cells of the respiratory system, the integumentary system, the urogenital system and the skin.

An interesting subclass of antimicrobial peptides is the one of plant defensins (Planta, 2002, 216, pp-193-202). Particularly interesting peptides among them are the following:
Hs-AFP1, corresponding to SEQ.ID.NO: 1
Rs-AFP2, corresponding to SEQ.ID.NO: 2
Ah-AMP1, corresponding to SEQ.ID.NO: 3
NmDef2, corresponding to SEQ.ID.NO: 4
Oh-DEF, corresponding to SEQ.ID.NO: 5
DefMT6, corresponding to SEQ.ID.NO: 6
AvBD1, corresponding to SEQ.ID.NO: 7
mDB14, corresponding to SEQ.ID.NO: 8
PsDef1, corresponding to SEQ.ID.NO: 9
Def-Tk, corresponding to SEQ.ID.NO: 10
Abf-2, corresponding to SEQ.ID.NO: 11
K7MPK0, corresponding to SEQ.ID.NO: 12
Defl.1, corresponding to SEQ.ID.NO: 13
OsDef8 corresponding to SEQ.ID.NO: 14
Termicin, corresponding to SEQ.ID.NO: 15

The peptides referred herein are per se known; for example, the peptide Hs-AFP1 is per se known from WO200472239, WO202186982 and WO2016205902; the peptide Rs-AFP2 is per se known e.g. from WO200109174 and WO200109175.

A subgroup of peptide preferred according to the invention is the subgroup consisting of Hs-AFP1, Rs-AFP2 or PsDef-1, which have the above-mentioned structures.

The fatty acids that can be used in the present composition are: pelargonic acid, crotonic acid, caproleic acid or mixtures thereof. The fatty acids used in the invention can either have or not have antimicrobial activity per se: in any case they synergically interact with the peptide, thereby causing an overall antimicrobial activity higher than the sum of the activities of the two components taken separately.

The present new combinations of defensins with fatty acids selected from the group consisting of crotonic acid, pelargonic acid, caproleic acid and mixtures thereof have shown a remarkably high level of synergic antimicrobial interaction (measured as FIC Index) against a large variety of target microorganisms, including fungi, Gram positive and Gram negative bacteria, inclusive of phytoplasma, thus conjugating the hardly conciliable effects of aspecificity and efficacy; the highest level of synergy is present when the defensins are combined with pelargonic acid, which represents an even more preferred combination. The obtained high levels of synergy are paralleled by a high level of antiinfective activity when applied on plant infections in open field, as confirmed by the experimental data included in this specification. A remarkable advantage of the combinations according to this sub-embodiment is their potent inhibitory activity against phytoplasma, a sub-class of bacteria responsible for hardly curable plant diseases, being resistant to conventional antibacterial agents; the level of activity is dramatically higher compared to that shown by the two separate products tested alone: this indicates that the synergism among these agents is especially amplified when the target microorganism is a phytoplasm; this is particularly unexpected because antimicrobial peptides and fatty acids are known to exert antimicrobial activity via interaction with the bacterial cell wall, a cellular component absent in phytoplasma. In the present sub-embodiment, any defensin can be used in combination with said crotonic, pelargonic and/or caproleic acid; examples of suitable defensins are: Hs-AFP1, corresponding to SEQ.ID.NO: 1; Rs-AFP2, corresponding to SEQ.ID.NO: 2; Ah-AMP1, corresponding to SEQ.ID.NO: 3; NmDef2, corresponding to SEQ.ID.NO: 4; Oh-DEF, corresponding to SEQ.ID.NO: 5; DefMT6, corresponding to SEQ.ID.NO: 6; AvBD1, corresponding to SEQ.ID.NO: 7;mDB14, corresponding to SEQ.ID.NO: 8; PsDefl, corresponding to SEQ.ID.NO: 9; Def-Tk, corresponding to SEQ.ID.NO: 10; Abf-2, corresponding to SEQ.ID.NO: 11; K7MPK0, corresponding to SEQ.ID.NO: 12; Def1.1, corresponding to SEQ.ID.NO: 13; OsDef8 corresponding to SEQ.ID.NO: 14; Termicin, corresponding to SEQ.ID.NO: 15. Particularly preferred are the combinations of crotonic acid, pelargonic acid and/or caproleic acid with one or more of said Hs-AFP1, Rs-AFP2 and PsDef1.

In all embodiments of the present invention, the peptides and the fatty acids can be combined with each other in all the possible proportions; preferably, neither of the two components is used in a weight ratio with respect to the other lower than 1:9. More preferably, the peptide (or their mixture, if more than one of them are used) is contained in a weight ratio with the fatty acid (or their mixture, if more than one of them are used) between 0.3:1 and 0.5:1; or alternatively between 0.5:1 and 1.5:1, for example in a 1:1 ratio.

In the present invention, the association of antimicrobial peptides with fatty acids obtains very high synergy levels, i.e. characterized by FIC index ≤ 0.7, preferably between 0.05 and 0.5. According to the standard literature, the FIC Index can be calculated with the following formula: $FIC index = MICA / MICa + MICB / MICb$ wherein "MICA and MICB" are the minimum inhibitory concentrations (MIC) of the two compounds A and B mixed with each other whereas "MICa and MICb" are the minimum inhibitory concentrations of the two components used singularly. FIC index < 1.0 means synergy of the compounds combined with each other; FIC index = 1.0 means that the compounds do not interact with each other; FIC index > 1.0 means antagonism of the compounds combined with each other.

The term "antimicrobial" used herein is to be understood as comprising the terms antifungal, antibacterial, antiviral and antiparasitic. Preferably, the antimicrobial treatment is an antifungal or antibacterial treatment.

For the purposes of the antifungal treatment, all the fungal species can be treated according to the invention. Among them, the species preferably recommended for the purposes of the present treatment are the following.

In the agronomic field: *Botrytis cinerea, Fusarium culmorum, Fusarium graminearum, Fusarium oxysporum, Fusarium solani, Stemphylium vesicarium, Scleratium rolfsii, Bipolaris sorokiniana, Sclerotinia sclerotiorum, Rhizoctonia solani, Zymoseptoria tritici, Cercospora beticola, Alternaria alternata, Venturia inequalis, Magnaporthe oryzae, Phytophtora infestans, Plasmopara viticola, Phakopsora pachyrhizi, Plasmopara viticola, Taphrina deformans, Uncinula necator, Erysiphe spp.* Particularly preferred for the purposes of said treatment are the species: *Botrytis cinerea, Fusarium culmorum, Fusarium graminearum, Phytophtora infestans, Alternaria alternata, Venturia inequalis.*

For the purposes of the antibacterial treatment, all the bacterial species, including phytoplasmas, can be treated in the agronomic field according to the invention. Among them, the species preferably recommended for the purposes of the present treatment are the following.

*Erwinia amylovora, Pseudomonas syringae, Xanthomonas campestris, Xanthomonas phaseoli, Xylella fastidiosa.* Phytoplasmas that may be mentioned are for example: *'Ca. Phytoplasma castaneae', Ca. Phytoplasma graminis', 'Ca. Phytoplasma japonicum', 'Ca. Phytoplasma lycopersici', 'Ca. Phytoplasma oryzae', 'Ca. Phytoplasma pruni', 'Ca. Phytoplasma pyri', 'Ca. Phytoplasma solani', 'Ca. Phytoplasma vitis'.*

In the present compositions, said peptides and fatty acids are suitably formulated with a carrier suitable for dispersing the composition on a substrate that needs it, for example an aerial part of a plant that can be treated by superficial or endotherapic application or an agricultural land. Said composition, comprising the carrier, may be indifferently solid, semisolid, liquid, etc. Solid compositions may, for example, be in the form of powders, pellets, granules, microcapsules, etc.; said solid compositions may be delivered as such or may be previously dispersed in a liquid medium before administration on the land or on the plant. Semisolid compositions may be in the form of creams, pastes, gels, hydrogels, and the like. Liquid compositions may be in the form of a solution, suspension, dispersion, colloid, emulsion, etc.; they may be administered as such or in the form of an aerosol or spray. In an optional embodiment of the invention, the composition is not in the form of liposomes. Depending on their physical form, on the nature of the active ingredients contained, and on the usage conditions, the present compositions may include, in addition to the above-mentioned peptides, fatty acids and carrier, further excipients and other co-formulation agents according to the prior art of the field; in particular, they can contain stabilizers, antioxidants, buffering agents, chelating agents, agents for controlling the pH for example buffer systems, isotonicity agents, emulsifiers, co-emulsifiers, thickeners, gelling agents, film-forming agents, lubricants, glidants, anti-aggregating agents, moisture absorbers, coloring agents, etc.

Depending on their physical form and on the treatment needs (plant type and/or land type), the present compositions may be administered as such or dispersed in water, in fertilizing solutions, in biostimulating solutions, etc. For the purposes of an effective treatment, it is useful that the composition is administered in such an amount to provide a dose of mixture [peptide+fatty acid]/hectare (ha) of land between 50 Kg and 0.1 Kg, preferably between 5 Kg and 1 Kg.

A further object of the present invention is the agronomic use of a composition as defined above, comprising one or more of said antimicrobial peptides and one or more of said fatty acids, as an antimicrobial, preferably antifungal, agent. The present compositions can be used for both a preventive and a curative purpose, depending on the needs. The present invention also describes the use of said one or more antimicrobial peptides and said one or more fatty acids as defined above, in a process of preparation of said antimicrobial, preferably antifungal, composition.

A further object of the present invention is a process for the preparation of said antimicrobial composition with high synergistic activity, said process comprising formulating with each other: one or more antimicrobial peptides as defined above, one or more fatty acids as defined above, and, optionally, a suitable carrier and/or co-formulation agents.

The present invention is now described by way of the following non-limiting examples.

### EXPERIMENTAL PART

### Example 1 - Evaluation of synergism

### GENERAL PROCEDURE

The antimicrobial activity was evaluated using the in-vitro susceptibility test with the microdilution method described in the Clinical and Laboratory Standard Institute protocols (M07- Methods for Dilution Antimicrobial Susceptibility Test for Bacteria That Grow Aerobically; M27 - Reference Method for Broth Dilution Antifungal Susceptibility Testing of Yeasts; Reference Method for Broth Dilution Antifungal Susceptibility Testing of Filamentous Fungi). The MIC (Minimum Inhibitory Concentration) for each compound of interest was determined by these methods.

The positive control of the antimicrobial activity was carried out using fluconazole (for fungi and yeasts) and ceftriaxone (for bacterial strains). The negative control (absence of the active compounds) was evaluated by observing the correct microbial growth of the species of interest.

The synergistic activity of the various compounds was evaluated in vitro with the microdilution method in 96-well plates. Samples of the compounds of interest were prepared by diluting said compounds in sterile physiological solution to a specific concentration of 4 times the previously determined MIC. Then, combinations at different concentrations of the antimicrobial peptides with the fatty acids were made and said samples were treated as described in the CLSI protocols.

The synergy between the peptides and the fatty acids according to the invention was evaluated by calculating the FIC index according to the following formula: $FIC index = MICA / MICa + MICB / MICb$ wherein "MICA and MICB" are the minimum inhibitory concentrations (MIC) of the two compounds A and B mixed with each other whereas "MICa and MICb" are the minimum inhibitory concentrations of the two components used singularly.

A value of FIC index < 1.0 means synergy of the compounds combined with each other; a value of FIC index = 1.0 means the absence of synergy of the compounds combined with each other; a value of FIC index > 1.0 means antagonism of the compounds combined with each other.

The experimental results obtained are shown in Table I and in Table II:

**Table I - Antifungal activity**

| ***Product A (Peptide)*** | ***Product B (Fatty acid)*** | ***FIC INDEX*** | ***Microorganism*** |
|---|---|---|---|
| | Pelargonic acid | 0.1-0.3 | *Botrytis* cinerea |
| | | | *Fusarium culmorum* |
| | | | *Fusarium graminearum* |
| Hs-AFP1, Rs-AFP2 or PsDef1 | | | *Bipolaris sorokiniana* |
| | | | *Sclerotinia sclerotiorum* |
| | Crotonic acid | 0.5-0.7 | |
| | | | *Rhizoctonia solani* |
| | | | *Zymoseptoria tritici* |
| | | | Cercospora *beticola* |
| | | | *Alternaria alternata* |
| | Caproleic acid | 0.5-0.7 | *Venturia inequalis* |
| | | | *Magnaporthe oryzae* |
| | | | *Phytophtora infestans* |
| | | | *Plasmopara viticola** |
| | | | *Phakopsora pachyrhizi** |

**Table II - Antibacterial activity**

| ***Product A (Peptide)*** | ***Product B (Fatty acid)*** | ***FIC INDEX*** | ***Microorganism*** |
|---|---|---|---|
| | Pelargonic acid | 0.1 - 0.3 | *E.coli* |
| Hs-AFP1, Rs-AFP2 or PsDef1 | Crotonic acid | 0.4-0.7 | *S.aureus* |
| | Caproleic acid | 0.4-0.7 | *P.aeruginosa* |

### Example 2

### Open field testing - Antifungal activity on Fusarium graminearum by mixtures of fatty acids and peptides

The antifungal activity of three peptides (SEQ.ID.NOs.: 1, 2 and 9), of the crotonic and pelargonic acid and of the mixtures of these fatty acids with the aforementioned peptides was evaluated on winter wheat and durum wheat suitably contaminated by *Fusarium graminearum* .

The peptides of SEQ.ID.NOs.: 1, 2 and 9 were dissolved in water at a concentration of 10% w / w. Aqueous solutions of crotonic and pelargonic acids were prepared at a concentration of 10% w / w. Aqueous solutions of peptides and solutions of fatty acids were mixed, in order to obtain six different mixtures at a concentration of 10% w / w of peptide and acid. The solutions were used at the dosages indicated in the table on both cultivars, after 2 days from the inoculation of the pathogenic strain *Fusarium graminearum.* Only one application was carried out at the time corresponding to the phenological scale BBCH *(Biologische Bundesanstalt, Bundessortenamt and CHemical industry)* 69-70 and the efficacy of the products was evaluated after 7 days for 3 weeks starting from the last check. The efficacy was assessed as incidence of leaves affected by the target pathogen compared to the untreated control: an increased % efficacy corresponds to a decreased number of leaves infested with the phytopathogen.

| **Sample** | **Concentration** | **Efficacy (%)** | | |
|---|---|---|---|---|
| | | **1° check** | **2° check** | **3° check** |
| Hs_AFP1 | 2 L/ha | 35 | 27,4 | 26,5 |
| Rs_AFP2 | 2 L/ha | 38 | 34 | 25,4 |
| Ps_Def1 | 2 L/ha | 30 | 27 | 32 |
| Pelargonic acid | 2 L/ha | 10 | 8 | 9 |
| Crotonic acid | 2 L/ha | 8 | 7 | 10 |
| Hs_AFP1 + Pelargonic acid | 1 L/ha + 1 L/ha | 84 | 82 | 78 |
| Rs_AFP2 + Pelargonic acid | 1 L/ha + 1 L/ha | 78 | 75 | 72 |
| Ps_Def1 + Pelargonic acid | 1 L/ha + 1 L/ha | 78 | 72 | 73 |
| Hs_AFP1 + Crotonic acid | 1 L/ha + 1 L/ha | 80 | 77 | 75 |
| Rs_AFP2 + Crotonic acid | 1 L/ha + 1 L/ha | 76 | 73 | 69 |
| Ps_Def1 + Crotonic acid | 1 L/ha + 1 L/ha | 73 | 67 | 64 |
| Reference commercial product | 1 L/ha | 88 | 84 | 73 |
| Untreated | 0 | 0 | 0 | 0 |

### Example 3

### Open filed testing on phytoplasma - Antibacterial activity on Ca. Phytoplasma vitis by mixtures of fatty acids and peptides

The activity on phytoplasma of three peptides (SEQ.ID.NO.: 1, 2 and 9), of crotonic and pelargonic acid and of the mixtures of these fatty acids with the aforementioned peptides were evaluated on *Pervinca rosea* suitably contaminated by *Ca.Phytoplasma vitis.*

The peptides of SEQ.ID.NO.: 1, 2 and 9 were dissolved in water at a concentration of 10% w/w. Aqueous solutions of crotonic and pelargonic acids were prepared at a concentration of 10% w/w. Aqueous solutions of peptides and solutions of fatty acids were mixed, in order to obtain six different mixtures at a concentration of 10% w/w of peptide and acid.

The evaluation of the efficacy of the products used was evaluated with ddPCR (Droplet Digital PCR) technique for the quantification of the genetic material (DNA and RNA) after 48 hours from exposure to endotherapy treatment. High %DNA and RNA values are related to reduced / no antibacterial activity. The solutions were used through endotherapic treatment at the dosages indicated in the following table:

| **Sample** | **Concentration** | **Efficacy (%)** | |
|---|---|---|---|
| | | **% DNA** | **% RNA** |
| Hs_AFP1 | 2 L/ha | 75 | 78 |
| Rs_AFP2 | 2 L/ha | 81 | 80 |
| Ps_Def1 | 2 L/ha | 82 | 80 |
| Crotonic acid | 2 L/ha | 76 | 84 |
| Pelargonic acid | 2 L/ha | 84 | 86 |
| Hs_AFP1 + crotonic acid | 1 L/ha + 1 L/ha | 42 | 48 |
| Rs_AFP2 + crotonic acid | 1 L/ha + 1 L/ha | 46 | 54 |
| Ps_Def1 + crotonic acid | 1 L/ha + 1 L/ha | 44 | 52 |
| Hs_AFP1 + pelargonic acid | 1 L/ha + 1 L/ha | 45 | 50 |
| Rs_AFP2 + pelargonic acid | 1 L/ha + 1 L/ha | 50 | 60 |
| Ps_Def1 + pelargonic acid | 1 L/ha + 1 L/ha | 52 | 52 |
| Untreated | 0 | 91 | 94 |

### SEQUENCE LISTING

**SEQ.ID.NO: 1** (Hs-AFP1) 
DGVKLCDVPSGTWSGHCGSSSKCSQQCKDREHFAYGGACHYQFPSVKC
FCKRQC
**SEQ.ID.NO: 2** (Rs-AFP2) 
QKLCQRPSGTWSGVCGNNNACKNQCIRLEKARHGSCNYVFPAHKCICYFP
C
**SEQ.ID.NO: 3** (Ah-AMP1) 
LCNERPSQTWSGNCGNTAHCDKQCQDWEKASHGACHKRENHWKCFCY
FNC
**SEQ.ID.NO: 4** (NmDef2)
   RECKAQGRHGTCFRDANCVQVCEKQAGWSHGDCRAQFKCKCIFEC
**SEQ.ID.NO: 5** (Oh-DEF) 
MLCKLSMFGAVLGVPACAIDCLPMGKTGGSCEGGVCGCRKLTFKILWDKK
FG
**SEQ.ID.NO: 6** (DefMT6);
   GFGCPLNQGACHNHCRSIKRRGGYCSGIIKQTCTCYRK
**SEQ.ID.NO: 7** (AvBD1)
   APGNKAECEREKGYCGFLKCSFPFVVSGKCSRFFFCCKNIW
**SEQ.ID.NO: 8** (mDB14);
   FLPKTLRKFFCRIRGGRCAVLNCLGKEEQIGRCSNSGRKCCRKKK
**SEQ.ID.NO: 9** (PsDef1); 
RMCKTPSGKFKGYCVNNTNCKNVCRTEGFPTGSCDFHVAGRKCYCYKPC
P
**SEQ.ID.NO: 10** (Def-Tk);
   SPAIWGCDSFLGYCRLACFAHEASVGQKECAEGMLCCIPNV
**SEQ.ID.NO: 11** (Abf-2); 
ADIDFSTCARMDVPILKKAAQGLCITSCSMQNCGTGSCKKRSGRPTCVCYR
CANGGGDIPLGAL
**SEQ.ID.NO: 12** (K7MPK0) 
SCPGLCAKRSKTWSGWCGSSNNCDKQCRTKEGATHGACHGNILKRACDC
YFKC
**SEQ.ID.NO: 13** (Defl.1);
   KTCENLADKYRGPCFSGCDTHCTTKENAVSGRCRDDFRCWCTKRC
**SEQ.ID.NO: 14** (OsDef8);
   RTCESQSHRFKGPCARKANCASVCNTEGFPDGYCHGVRRRCMCTKPCP
**SEQ.ID.NO: 15** (Termicin)
   ACNFQSCWATCQAQHSIYFRRAFCDRSQCKCVFVRG
**SEQ.ID.NO: 16** (Ay-AMP)
   VGECVRGRCPSGMCCSQFGYCGKGPKYCGR
**SEQ.ID.NO: 17** (Ee-CBP)
   QQCGRQAGNRRCANNLCCSQYGYCGRTNEYCCTSQGCQSQCRRCG
**SEQ.ID.NO: 18** (StSN1) 
GSNFCDSKCKLRCSKAGLADRCLKYCGICCEECKCVPSGTYGNKHECPCY
RDKKNSKGKSKCP
**SEQ.ID.NO: 19** (McAMP1)
   AKCIKNGKGCREDQGPPFCCSGFCYRQVGWARGYCKNR
**SEQ.ID.NO: 20** (VtA3)
   KSCCPNTTGRNIYNACRLTGAPRPTCAKLSGCKIISGSTCPSDYPK

## Claims

1. A composition comprising at least one antimicrobial peptide selected from the class of defensins and at least one fatty acid selected from pelargonic acid, crotonic acid, caproleic acid, or mixtures thereof.

2. The composition according to claim 1, wherein said defensins are selected from: Hs-AFP1, corresponding to SEQ.ID.NO: 1; Rs-AFP2, corresponding to SEQ.ID.NO: 2; Ah-AMP1, corresponding to SEQ.ID.NO: 3; NmDef2, corresponding to SEQ.ID.NO: 4; Oh-DEF, corresponding to SEQ.ID.NO: 5; DefMT6, corresponding to SEQ.ID.NO: 6; AvBD1, corresponding to SEQ.ID.NO: 7; mDB14, corresponding to SEQ.ID.NO: 8; PsDefl, corresponding to SEQ.ID.NO: 9; Def-Tk, corresponding to SEQ.ID.NO: 10; Abf-2, corresponding to SEQ.ID.NO: 11; K7MPK0, corresponding to SEQ.ID.NO: 12; Defl.1, corresponding to SEQ.ID.NO: 13; OsDef8 corresponding to SEQ.ID.NO: 14; Termicin, corresponding to SEQ.ID.NO: 15.

3. Use of the composition according to claims 1-2 as antimicrobial agent in the agronomic field.

4. Use according to claim 3 in the treatment or prevention of contaminations by fungi and/or bacteria, inclusive phytoplasma.

5. Use according to claim 4, wherein said fungi are selected from: *Botrytis cinerea, Fusarium culmorum, Fusarium graminearum, Fusarium oxysporum, Fusarium solani, Stemphylium vesicarium, Scleratium rolfsii, Bipolaris sorokiniana, Sclerotinia sclerotiorum, Rhizoctonia solani, Zymoseptoria tritici, Cercospora beticola, Alternaria alternata, Venturia inequalis, Magnaporthe oryzae, Phytophtora infestans, Plasmopara viticola, Phakopsora pachyrhizi, Plasmopara viticola, Taphrina deformans, Uncinula necator, Erysiphe spp., Candida albicans, Aspergillus fumigatus, Cryptococcus neoformans, Malassezia furfur, Trichosporon spp, Histoplasma capsulatum, Blastomyces dermatitidis, Coccidioides immitis.*

6. Use according to claim 4, wherein said bacteria are selected from *Erwinia amylovora, Pseudomonas syringae, Xanthomonas campestris, Xanthomonas phaseoli, Xylella fastidiosa, Pseudomonas aeruginosa, Staphylococcus aureus, Escherichia coli, Campylobacter jejuni, Bacillus cereus, Listeria monocytogenes, Salmonella typhimurium, Clostridium perfringens,* or phytoplasmas *'Ca. Phytoplasma castaneae', Ca. Phytoplasma graminis', 'Ca. Phytoplasma japonicum', 'Ca. Phytoplasma lycopersici', 'Ca. Phytoplasma oryzae', 'Ca. Phytoplasma pruni', 'Ca. Phytoplasma pyri', 'Ca. Phytoplasma solani', 'Ca. Phytoplasma vitis'.*

7. A process for the preparation of the antimicrobial composition described in claims 1-2, comprising formulating with each other: one or more of said antimicrobial peptides, one or more of said fatty acids, and, optionally, a suitable carrier and/or suitable co-formulation agents.

## Patentansprüche

1. Zusammensetzung, die mindestens ein antimikrobielles Peptid ausgewählt aus der Klasse der Defensine und mindestens eine Fettsäure ausgewählt aus Pelargonsäure, Crotonsäure, Caproleinsäure oder Mischungen davon enthält.

2. Zusammensetzung nach Anspruch 1, wobei die Defensine ausgewählt sind aus: Hs-AFPl, entsprechend SEQ.ID.NO: 1; Rs-AFP2, entsprechend SEQ.ID.NO: 2; Ah-AMP1, entsprechend SEQ.ID.NO: 3; NmDef2, entsprechend SEQ.ID.NO: 4; Oh-DEF, entsprechend SEQ.ID.NO: 5; DefMT6, entsprechend SEQ.ID.NO: 6; AvBD1, entsprechend SEQ.ID.NO: 7; mDB14, entsprechend SEQ.ID.NO: 8; PsDefl, entsprechend SEQ.ID.NO: 9; Def-Tk, entsprechend SEQ.ID.NO: 10; Abf-2, entsprechend SEQ.ID.NO: 11; K7MPK0, entsprechend SEQ.ID.NO: 12; Def 1.1, entsprechend SEQ.ID.NO: 13; OsDef8, entsprechend SEQ.ID.NO: 14; Termicin, entsprechend SEQ.ID.NO: 15.

3. Verwendung der Zusammensetzung gemäß den Ansprüchen 1-2 als antimikrobielles Mittel im agrarwirtschaftlichen Bereich.

4. Verwendung gemäß Anspruch 3 zur Behandlung oder Vorbeugung von Kontaminationen durch Pilze und/oder Bakterien, einschließlich Phytoplasmen.

5. Verwendung gemäß Anspruch 4, wobei die Pilze ausgewählt sind aus: *Botrytis cinerea, Fusarium culmorum, Fusarium graminearum, Fusarium oxysporum, Fusarium solani, Stemphylium vesicarium, Scleratium rolfsii, Bipolaris sorokiniana, Sclerotinia sclerotiorum, Rhizoctonia solani, Zymoseptoria tritici, Cercospora beticola, Alternaria alternata, Venturia inequalis, Magnaporthe oryzae, Phytophtora infestans, Plasmopara viticola, Phakopsora pachyrhizi, Plasmopara viticola, Taphrina deformans, Uncinula necator, Erysiphe spp., Candida albicans, Aspergillus fumigatus, Cryptococcus neoformans, Malassezia furfur, Trichosporon spp., Histoplasma capsulatum, Blastomyces dermatitidis, Coccidioides immitis.*

6. Verwendung nach Anspruch 4, wobei die Bakterien ausgewählt sind aus *Erwinia amylovora, Pseudomonas syringae, Xanthomonas campestris, Xanthomonas phaseoli, Xylella fastidiosa, Pseudomonas aeruginosa, Staphylococcus aureus, Escherichia coli, Campylobacter jejuni, Bacillus cereus, Listeria monocytogenes, Salmonella typhimurium, Clostridium perfringens* oder den *Phytoplasmen "Ca. Phytoplasma castaneae* ", *"Ca. Phytoplasma graminis* ", *"Ca. Phytoplasma japonicum* ", *"Ca. Phytoplasma lycopersici* ", *"Ca. Phytoplasma oryzae* ",
*"Ca. Phytoplasma pruni* ", *"Ca. Phytoplasma pyri", "Ca. Phytoplasma solani* ", *"Ca. Phytoplasma vitis ".*

7. Verfahren zur Herstellung der in den Ansprüchen 1-2 beschriebenen antimikrobiellen Zusammensetzung, das das Zusammenformulieren folgender Bestandteile umfasst: eines oder mehrerer der genannten antimikrobiellen Peptide, einer oder mehrerer der genannten Fettsäuren und gegebenenfalls eines geeigneten Trägers und/oder geeigneter Co-Formulierungsmittel.

## Revendications

1. Composition comprenant au moins un peptide antimicrobien choisi parmi la classe des défensines et au moins un acide gras choisi parmi l'acide pélargonique, l'acide crotonique, l'acide caproléique, ou leurs mélanges.

2. Composition selon la revendication 1, dans laquelle lesdites défensines sont choisies parmi : Hs-AFP1, correspondant à SEQ.ID.NO: 1 ; Rs-AFP2, correspondant à SEQ.ID.NO: 2 ; Ah-AMP1, correspondant à SEQ.ID.NO: 3 ; NmDef2, correspondant à SEQ.ID.NO: 4 ; Oh-DEF, correspondant à SEQ.ID.NO: 5 ; DefMT6, correspondant à SEQ.ID.NO: 6 ; AvBD1, correspondant à SEQ.ID.NO: 7 ; mDB14, correspondant à SEQ.ID.NO: 8 ; PsDefl, correspondant à SEQ.ID.NO: 9 ; Def-Tk, correspondant à SEQ.ID.NO: 10 ; Abf-2, correspondant à SEQ.ID.NO: 11 ; K7MPK0, correspondant à SEQ.ID.NO: 12 ; Def1.1, correspondant à SEQ.ID.NO: 13 ; OsDef8 correspondant à SEQ.ID.NO: 14 ; Termicin, correspondant à SEQ.ID.NO: 15.

3. Utilisation de la composition selon les revendications 1 à 2 comme agent antimicrobien dans le domaine agronomique.

4. Utilisation selon la revendication 3 dans le traitement ou la prévention de contaminations par des champignons et/ou des bactéries, y compris des phytoplasmes.

5. Utilisation selon la revendication 4, dans laquelle lesdits champignons sont choisis parmi *: Botrytis cinerea, Fusarium culmorum, Fusarium graminearum, Fusarium oxysporum, Fusarium solani, Stemphylium vesicarium, Scleratium rolfsii, Bipolaris sorokiniana, Sclerotinia sclerotiorum, Rhizoctonia solani, Zymoseptoria tritici, Cercospora beticola, Alternaria alternata, Venturia inaequalis, Magnaporthe oryzae, Phytophthora infestans, Plasmopara viticola, Phakopsora pachyrhizi, Plasmopara viticola, Taphrina deformans, Uncinula necator, Erysiphe spp., Candida albicans, Aspergillus fumigatus, Cryptococcus neoformans, Malassezia furfur, Trichosporon spp, Histoplasma capsulatum, Blastomyces dermatitidis, Coccidioides immitis.*

6. Utilisation selon la revendication 4, dans laquelle lesdites bactéries sont choisies parmi *Erwinia amylovora, Pseudomonas syringae, Xanthomonas campestris, Xanthomonas phaseoli, Xylella fastidiosa, Pseudomonas aeruginosa, Staphylococcus aureus, Escherichia coli, Campylobacter jejuni, Bacillus cereus, Listeria monocytogenes, Salmonella typhimurium, Clostridium perfringens,* ou les phytoplasmes « *Ca. Phytoplasma castaneae », « Ca. Phytoplasma graminis », « Ca. Phytoplasma japonicum », « Ca. Phytoplasma lycopersici », « Ca. Phytoplasma oryzae », « Ca. Phytoplasma pruni », « Ca. Phytoplasma pyri », « Ca. Phytoplasma solani », « Ca. Phytoplasma vitis ».*

7. Procédé de préparation de la composition antimicrobienne décrite dans les revendications 1 à 2, comprenant la formulation, les uns avec les autres, d'un ou plusieurs desdits peptides antimicrobiens, d'un ou plusieurs desdits acides gras, et, éventuellement, d'un support approprié et/ou d'agents de coformulation appropriés.
